# EUROPEAN PATENT APPLICATION

(11) **EP 2 620 177 A1**
(43) Date of publication of application: **31.07.2013**
(21) Application number: 11826445.6
(22) Date of filing: 19.09.2011
(51) Int. Cl.: A61M 37/00

(54) **SHOCK-ABSORBING NEEDLE BAR FOR TATTOO MACHINES**

(30) Priority: 21.09.2010 ES 201001237 P
(71) Applicant: Bonatti Andrada, Richard, 08830 Sant Boi De Llobregat (ES)
(72) Inventor: Bonatti Andrada, Richard, 08830 Sant Boi De Llobregat (ES)
(74) Representative: ZBM Patents
(86) International application number: PCT/ES2011/070653
(87) International publication number: WO 2012/038571

(57) **Abstract**

A needle-holding for a tattooing machine that comprises two bar segments 1A and 1 B, separated but substantially aligned, an elastic element 3 arranged longitudinally between the two segments, a rigid tube 2 that houses the elastic elements and receives a part of each segment in its interior, a shrinkable sheath 5A arranged on the bar segment 1A and a shrinkable sheath 5B arranged on the bar segment 1 B, the separation between each shrinkable sheath and each end of the rigid tube being longer than the possible separation between each segment and the elastic element, and a shrinkable sleeve 4 that covers a portion of each shrinkable sheath and joins them to the rigid tube.

## Description

The invention is related to a needle-holding bar for a tattoing machine, to a method of manufacturing such a bar and to a method of tattooing.

### STATE OF THE ART

Tattooing machines are portable devices equipped with a needle having a reciprocating motion that enters and exits the skin and inserts pigments (for example, indelible ink) underneath the epidermis. Such a needle, or needles (there may be several), is mounted on holding a bar the other end of which has a hook shape and is engaged to the reciprocating motion mechanism.

The bars are made of steel, and consequently are not very flexible, and sometimes they drive the needle too deeply underneath the skin, drawing blood, which, besides being painful, complicates the making of the tattoo and impairs its quality.

### SUMMARY OF THE INVENTION

It is an objective of the present invention to diminish the pain as well as the bleeding during the tattooing, and also to improve the ink penetration and to accelerate healing.

In accordance with one aspect of the invention, the needle-holding bar comprises two separated but substantially aligned bar segments, an elastic element arranged longitudinally between the two segments, a rigid tube that houses the elastic element and receives a portion of each segment in its interior, and a shrinkable sleeve that covers the tube and a portion of each segment and joins these three elements.

In this way, the elastic element interposed in the bar provides a buffering system that actuates on the incidence of the needle at the skin, limiting and softening the penetration of the needle in the layers of the skin, the dermis and epidermis. This improves the ink penetration and accelerates the healing, causing less pain in the process as there is less trauma.

The rigid tube serves for keeping the two segments of the bar aligned along its axial direction, and the shrinkable sleeve serves to hold all the pieces longitudinally in place and keep the bar from being divided.

In an embodiment the elastic element is separated from each bar segment, thus allowing the top bar segment, which receives the reciprocating motion, a certain degree of unproductive linear motion before the elastic element provides the buffering.

In an embodiment the elastic element is substantially cylindrical, the concept of "cylinder" being understood to include different cross-sectional shapes, such as a circle or square.

The elastic element is preferably made of rubber, which is the most common and cheap elastic material.

The rigid tube is preferably made of PVC, which is a commonly used cheap and rigid plastic.

In an embodiment the shrinkable sleeve is a rubber element, although it is preferably made of a heat-shrinkable material.

Advantageously the bar comprises a shrinkable sheath arranged on one of the bar segments and a shrinkable sheath arranged on the other bar segment, the separation between each shrinkable sheath and each end of rigid tube being longer than the possible separation between each segment and the elastic element. These sheaths serve as an abutment for each bar segment against the rigid tube, so that the elastic element does not become too compressed.

In a manner analogous to the above one, the shrinkable sleeve covers the rigid tube and a portion of each shrinkable sheath, joining them to said tube to hold all the pieces in place longitudinally and to keep the bar from becoming divided.

In an embodiment the shrinkable sheath is made of a heat-shrinkable material.

In accordance with another aspect of the invention, a method of manufacturing the needle-holding bar of the invention comprises the steps of:
- cutting a conventional needle-holding bar into two bar segments;
- introducing the elastic element into the rigid tube;
- introducing the bar segments into the rigid tube, one through one end of the tube and the other through the other end;
- covering the assembly, although without spanning the complete bar, with a heat shrinkable tubular strip (sleeve);
- heating the bar in order for the heat shrinkable sleeve to shrink on the whole assembly and to hold it together.

Preferably a small separation is left inside the rigid tube between each bar segment and the elastic element in order to avoid the risk of the elastic element becoming permanently compressed or compressed more than it should.

In accordance with yet another aspect of the invention, a method of manufacturing the needle-holding bar of the invention comprises the steps of:
- cutting a conventional needle-holding bar into two bar segments;
- sheathing one of the bar segments with a heat-shrinkable sheath and the other bar segment with another heat-shrinkable sheath;
- introducing the elastic element into the rigid tube;
- introducing the bar segments into the rigid tube, one through one end of the tube and the other through the other end, without the heat-shrinkable sheaths abutting against the tube;
- covering the assembly, although without spanning the complete bar, with a heat-shrinkable tubular strip (sleeve);
- heating the bar in order for the heat shrinkable sleeve to shrink on the whole assembly and to hold it together.

Preferably a separation is left between each bar segment and the elastic element inside the rigid tube, and a longer separation is left between each heat-shrinkable sheath and the rigid tube, so that the sheaths serve as an abutment.

In accordance with another aspect of the invention, a method for tattooing employs the needle-holding bar of the invention.

### BRIEF DESCRIPTION OF THE DRAWINGS

Some particular embodiments of the present invention will be described in the following, only by way of non-limiting example, with reference to the appended drawings, in which:
figure 1 is a side view of the divided bar;
figure 2 is a perspective view of the rigid tube;
figures 3, 4 and 5 are side views of an embodiment of the bar; and
figures 6, 7, 8 and 9 are side views of another embodiment of the bar.

### DESCRIPTION OF PREFERRED EMBODIMENTS

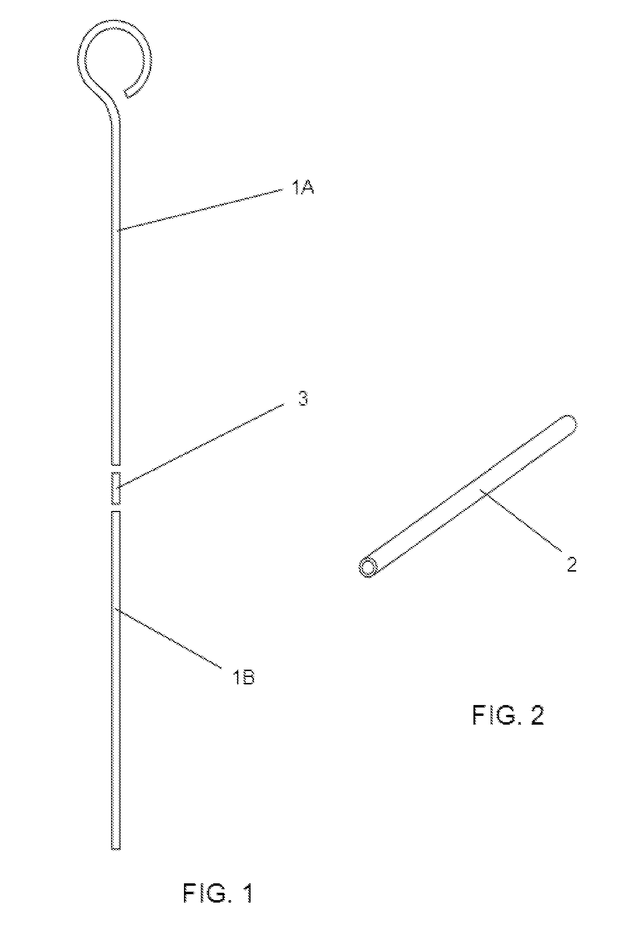

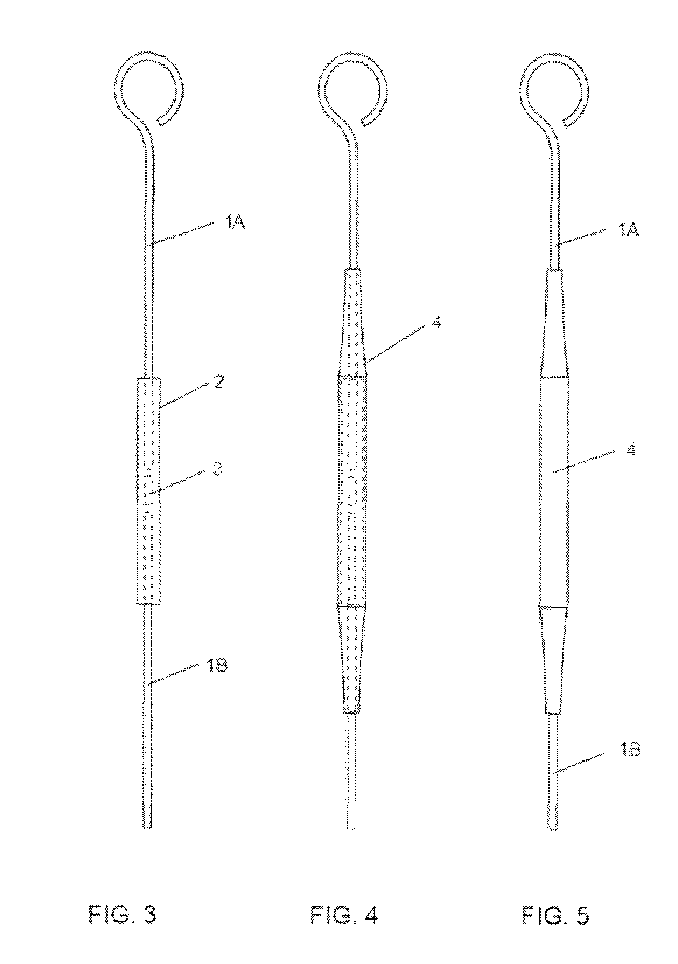

Figure 1 shows a conventional bar cut into two pieces or segments 1A and 1 B, with a cylindrical rubber element 3 between them. Figure 2 shows a rigid plastic tube 2 into which the rubber element 3 and the segments 1A and 1 B are introduced, as shown in figure 3, said elements being aligned and the rubber element being placed between them. The rigid tube 2 prevents the bar from flexing laterally but allows the axial motion of the two bar segments.

Figure 4 shows the bar of figure 3 covered with heat-shrinkable plastic sleeve 4, which when heated shrinks on the segments 1A and 1 B and the tube 2 (the sleeve 4 may be, for example, a shrinkable rubber element), leaving them joined in order to hold them in place longitudinally (that is, in the axial direction), while having a certain longitudinal flexibility. Segments 1A and 1 B may have a small relative longitudinal motion (of entry and exit of tube 4, compressing the rubber element 3 or separating themselves from it), but the shrinkable sheath keeps them joined together once it has been shrunk.

Figure 5 is like figure 4 but only with visible lines (the dotted lines in the other figures represent unseen lines).

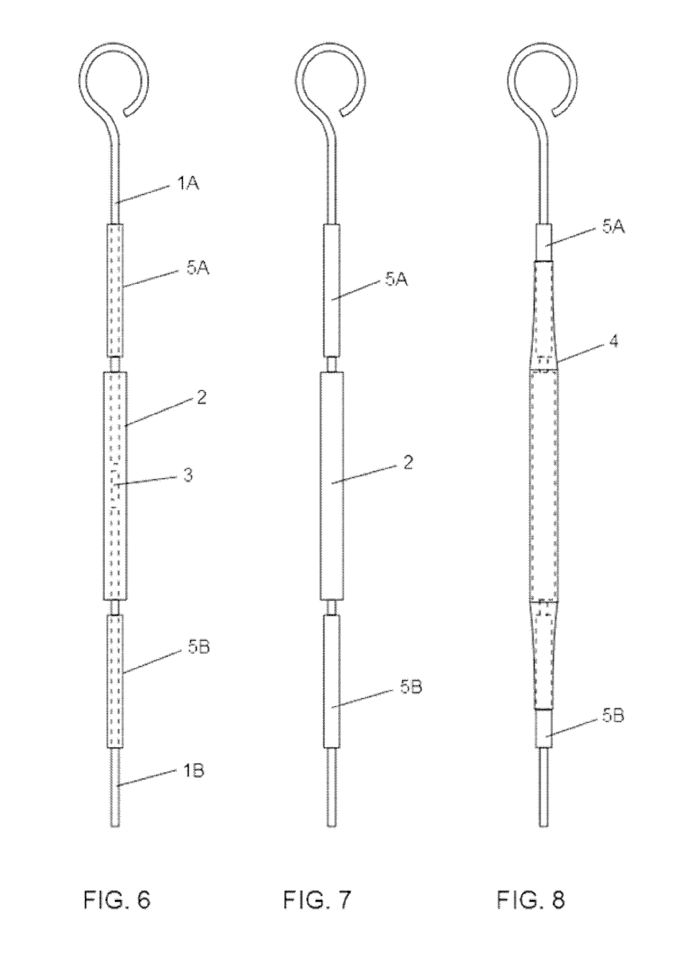

In another embodiment, a shrinkable strip (for example, heat shrinkable) 5A and 5B, respectively, is applied on each segment of bar 1A and 1 B, as shown in figures 6 and 7. After being heated, these strips constitute sheaths that act as abutments for the entry motion of the segments 1A and 1 B into the tube 2, which prevents the rubber element 3 from being excessively compressed. For this reason, the separation between each sheath 5A and 5B, and the tube 2 is greater than the separation between each segment 1A and 1 B and the rubber element 3 (which could be null).

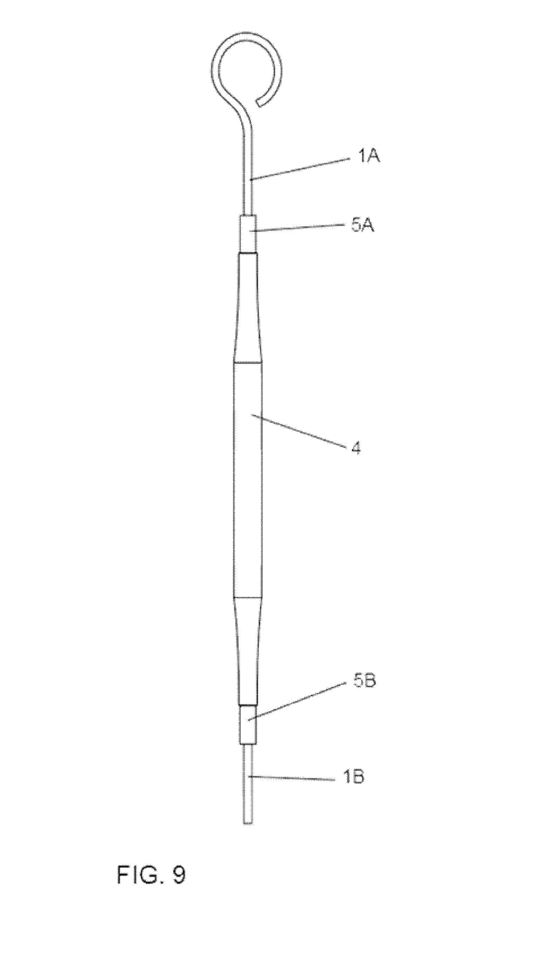

Just as in the first embodiment, the shrinkable sleeve 4 is also applied on the bar having the shrinkable sheaths 5A and 5B, in this case covering the tube 2 and said shrinkable sheaths for the same purpose of keeping the assembly joined together (figure 8). Figure 9 is like figure 8 but with the lines visible.

The bar segments 1 A and 1 B may have a length of approximately 5.5 cm and a diameter of approximately 1.5 mm; the rigid tube 2 a length of approximately 4 cm and a diameter of approximately 2 mm; the rubber cylinder 3 a length of approximately 0.5 cm and a diameter of approximately 1.5 mm; the shrinkable sheaths 5A and 5B a length of approximately 3 cm and a diameter of approximately 2 mm; and the shrinkable sleeve 4 a length of approximately 6.5 cm and a diameter of approximately 2.5 mm.

Although only particular embodiments of the invention have been shown and described in the present specification, the skilled man will be able to introduce modifications and substitute any technical features thereof with others that are technically equivalent, depending on the particular requirements of each case, without departing from the scope of protection defined by the appended claims.

## Claims

1. Needle-holding bar for a tattooing machine, **characterised by** comprising two bar segments (1A, 1 B), separated but substantially aligned, an elastic element (3) arranged longitudinally between the two segments, a rigid tube (2) that houses the elastic element and receives a portion of each segment in its interior, and a shrinkable sleeve (4) that covers the tube and a portion of each segment, and which joins these three elements.

2. Bar according to claim 1, wherein the elastic element is separated from each bar segment.

3. Bar according to claim 1 or 2, wherein the elastic element is substantially cylindrical.

4. Bar according to any of the previous claims, wherein the elastic element is made of rubber.

5. Bar according to any of the previous claims, wherein the rigid tube is made of PVC.

6. Bar according to any of the previous claims, wherein the shrinkable sleeve is made of rubber.

7. Bar according to any of the previous claims, wherein the shrinkable sleeve is made of a heat-shrinkable material.

8. Bar according to any of the previous claims, comprising a shrinkable sheath (5A) arranged on one bar segment (1A) and a shrinkable sheath (5B) arranged on the other bar segment (1B), the separation between each shrinkable sheath and each end of the rigid tube (2) being longer than the possible separation between each segment and the elastic element (3).

9. Bar according to claim 8, wherein the shrinkable sleeve covers a portion of each shrinkable sheath and joins them to the rigid tube.

10. Bar according to claim 8 or 9, wherein the shrinkable sheath is made of a heat shrinkable material.

11. Method of manufacturing a needle-holding bar according to any of claims 1 to 7, comprising the steps of:
- cutting a conventional needle-holding bar into two bar segments (1A, 1 B);
- introducing the elastic element (3) into the rigid tube (2);
- introducing the bar segments into the rigid tube, one through one end of the tube and the other through the other end;
- covering the assembly, although without spanning the complete bar, with a heat-shrinkable sleeve (4);
- heating the bar in order for the heat shrinkable sleeve to shrink on the whole assembly and to hold it together.

12. Method according to claim 11, in which a separation is left between each bar segment and the elastic element inside the rigid tube.

13. Method of manufacturing a needle-holding bar according to any of claims 8 to 10, comprising the steps of:
- cutting a conventional needle-holding bar into two bar segments (1A, 1 B);
- sheathing one of the bar segments (1A) with a heat-shrinkable sheath (5A) and the other bar segment (1 B) with another heat-shrinkable sheath (5B);
- introducing the elastic element (3) into the rigid tube (2);
- introducing the bar segments into the rigid tube, one through one end of the tube and the other through the other end, without the heat-shrinkable sheaths abutting against the tube;
- covering the assembly, although without spanning the complete bar, with a heat-shrinkable sleeve (4);
- heating the bar in order for the heat-shrinkable sleeve to shrink on the whole assembly and to hold it together.

14. Method according to claim 13, in which a separation is left inside the rigid tube between each bar segment and the elastic element, and a longer separation is left between each heat-shrinkable sheath and the rigid tube.

15. Method of tattooing, in which the needle-holding bar is a bar as defined in any of claims 1 to 10.

## Amended claims

### Amended claims under Art. 19.1 PCT

**1.** Needle-holding bar for a tattooing machine, comprising two bar segments (1A, 1 B), separated but substantially aligned, an elastic element (3) arranged longitudinally between the two segments, and a rigid tube (2) that houses the elastic element and receives a portion of each segment in its interior, **characterized by** further comprising a shrinkable sleeve (4) that covers the tube and a portion of each segment, and which joins these three elements.

**2.** Bar according to claim 1, wherein the elastic element is separated from each bar segment.

**3.** Bar according to claim 1 or 2, wherein the elastic element is substantially cylindrical.

**4.** Bar according to any of the previous claims, wherein the elastic element is made of rubber.

**5.** Bar according to any of the previous claims, wherein the rigid tube is made of PVC.

**6.** Bar according to any of the previous claims, wherein the shrinkable sleeve is made of rubber.

**7.** Bar according to any of the previous claims, wherein the shrinkable sleeve is made of a heat-shrinkable material.

**8.** Bar according to any of the previous claims, comprising a shrinkable sheath (5A) arranged on one bar segment (1A) and a shrinkable sheath (5B) arranged on the other bar segment (1B), the separation between each shrinkable sheath and each end of the rigid tube (2) being longer than the possible separation between each segment and the elastic element (3).

**9.** Bar according to claim 8, wherein the shrinkable sleeve covers a portion of each shrinkable sheath and joins them to the rigid tube.

**10.** Bar according to claim 8 or 9, wherein the shrinkable sheath is made of a heat shrinkable material.

**11.** Method of manufacturing a needle-holding bar according to any of claims 1 to 7, comprising the steps of:
- cutting a conventional needle-holding bar into two bar segments (1A, 1 B);
- introducing the elastic element (3) into the rigid tube (2);
- introducing the bar segments into the rigid tube, one through one end of the tube and the other through the other end;
- covering the assembly, although without spanning the complete bar, with a heat-shrinkable sleeve (4);
- heating the bar in order for the heat shrinkable sleeve to shrink on the whole assembly and to hold it together.

**12.** Method according to claim 11, in which a separation is left between each bar segment and the elastic element inside the rigid tube.

**13.** Method of manufacturing a needle-holding bar according to any of claims 8 to 10, comprising the steps of:
- cutting a conventional needle-holding bar into two bar segments (1A, 1 B);
- sheathing one of the bar segments (1A) with a heat-shrinkable sheath (5A) and the other bar segment (1 B) with another heat-shrinkable sheath (5B);
- introducing the elastic element (3) into the rigid tube (2);
- introducing the bar segments into the rigid tube, one through one end of the tube and the other through the other end, without the heat-shrinkable sheaths abutting against the tube;
- covering the assembly, although without spanning the complete bar, with a heat-shrinkable sleeve (4);
- heating the bar in order for the heat-shrinkable sleeve to shrink on the whole assembly and to hold it together.

**14.** Method according to claim 13, in which a separation is left inside the rigid tube between each bar segment and the elastic element, and a longer separation is left between each heat-shrinkable sheath and the rigid tube.

**15.** Method of tattooing, in which the needle-holding bar is a bar as defined in any of claims 1 to 10.
